Europäisches Patentamt

European Patent Office

Office européen des brevets

(19)

(11) Publication number: **0 234 002 B1**

## EUROPEAN PATENT SPECIFICATION

(12)

(45) Date of publication of patent specification :
**09.01.91 Bulletin 91/02**

(51) Int. Cl.[5] : **C07C 17/34,** C07C 21/18,
B01J 27/132

(21) Application number : **86115828.5**

(22) Date of filing : **14.11.86**

(54) Process for converting a 1,1,1-trifluoroalkane into a 1,1-difluoroalkene.

(30) Priority : **22.01.86 US 821316**

(43) Date of publication of application :
**02.09.87 Bulletin 87/36**

(45) Publication of the grant of the patent :
**09.01.91 Bulletin 91/02**

(84) Designated Contracting States :
**BE DE FR GB IT NL**

(56) References cited :
**EP-A- 0 036 123
US-A- 2 480 560
US-A- 2 745 886
US-A- 3 456 025**

(56) References cited :
J. MARCH "Advanced Organic Chemistry",
2nd edition, 1977, pages 935-936, McGraw-Hill
International Book Co., London, GB;
PATENT ABSTRACTS OF JAPAN, vol. 3, no.
152 (C-67), 14th December 1979, page 121 C
67; & JP - A - 54 130 507 (DAIKIN KOGYO)
10-09-1979

(73) Proprietor : **ATOCHEM NORTH AMERICA, INC.
(a Pennsylvania corp.)
Three Parkway
Philadelphia Pennsylvania 19102 (US)**

(72) Inventor : **Elsheikh, Maher Y.
784 North Wayne Avenue
Wayne Pennsylvania 19078 (US)**

(74) Representative : **Kraus, Walter, Dr. et al
Patentanwälte Kraus, Weisert & Partner
Thomas-Wimmer-Ring 15
D-8000 München 22 (DE)**

## Description

This invention relates generally to the catalytic dehydrofluorination of fluorocarbons and more specifically to the catalytic dehydrofluorination of a 1,1,1-trifluoroalkane using an inorganic chromium oxyfluoride as a catalyst.

The dehydrofluorination of 1,1,1-trifluoroethane to 1,1-difluoroethylene at 400°C using NiO on alumina, $Fe_2O_3$, ZnO, and Ag on alumina has been disclosed by Walker et al., J. org. Chem., (30), p 3284 (1965). Conversions of up to 63.4% were reported, depending upon the catalyst used. JP-A-54-130507 (1979) discloses the conversion of 1,1,1trifluoroethane into 1,1-difluoroethylene (vinylidene fluoride) using an inorganic chromium III ($Cr^{+++}$) compound as a catalyst at temperatures of from about 350 to 500°C and states that the catalyst is almost unchanged in the reaction. Prior to use, the catalyst is stabilized by treatment for 1 to 5 hours in a HF atmosphere at about 450°C (the preparation of oxyfluoride catalysts from hydrated chromium fluoride by a similar procedure for use in fluorination reactions is described in US-A-2,745,886). At reaction temperatures of 375 to 417°C, using various chromium III catalysts, conversion rates of 10% to 52.7% are reported for the dehydrofluorination. A slight amount of $CO_2$ in addition to the expected HF and vinylidene fluoride is produced.

Patent Abstracts of Japan, Vol. 3, No. 152 (C-67), December 14, 1979 (JR 34-13 05 07), discloses the preparation of vinylidene fluoride by carrying out dehydrofluorination of 1,1,1-trifluoroethane in the presence of a Cr (III) compound, as a catalyst.

US-A-3 456 025 describes a process of dehydrofluorinating a trifluoroalkane by contacting said trifluoroalkane with a fluorided alumina catalyst under dehydrofluorination conditions.

It now has been found an improved process for preparing 1,1-difluoroalkenes such as vinylidene fluoride using an inorganic chromium catalyst which provides 100% conversions to the desired product for extended periods of time. Periodic catalyst treatment with hot oxygen is included to return the conversion to 100% when it falls below a selected rate.

In accordance with this invention there is provided a process for making a 1,1-difluoroalkene, which is characterized by flowing a gaseous 1,1,1-trifluoroalkane of the formula $RCH_2\text{-}CF_3$, where R is selected from hydrogen, methyl, isopropyl, t-butyl, cyclohexyl, vinyl, allyl and phenyl, through a catalyst bed which includes an inorganic chromium oxyfluoride obtained by treating chromium trifluoride hydrate powder with oxygen gas at a temperature of 120 to 600°C for 1 to 5 hours and optionally with hydrogen fluoride at a temperature of 400 to 450°C for 2 to 4 hours, at a reaction temperature above 430°C to form a 1,1-difluoroalkene of the formula $RCH=CF_2$

and HF as reaction products, and periodically reactivating the catalyst by interrupting the flow of 1,1,1-trifluoroalkane and passing an oxygen gas through said catalyst bed at a temperature of from 400 to 600°C for at least about 1/2 hour.

Figure 1 is a schematic diagram illustrating a system for carrying out the process of the invention.

Figure 2 is a graph illustrating the conversion rates of an embodiment of the process invention.

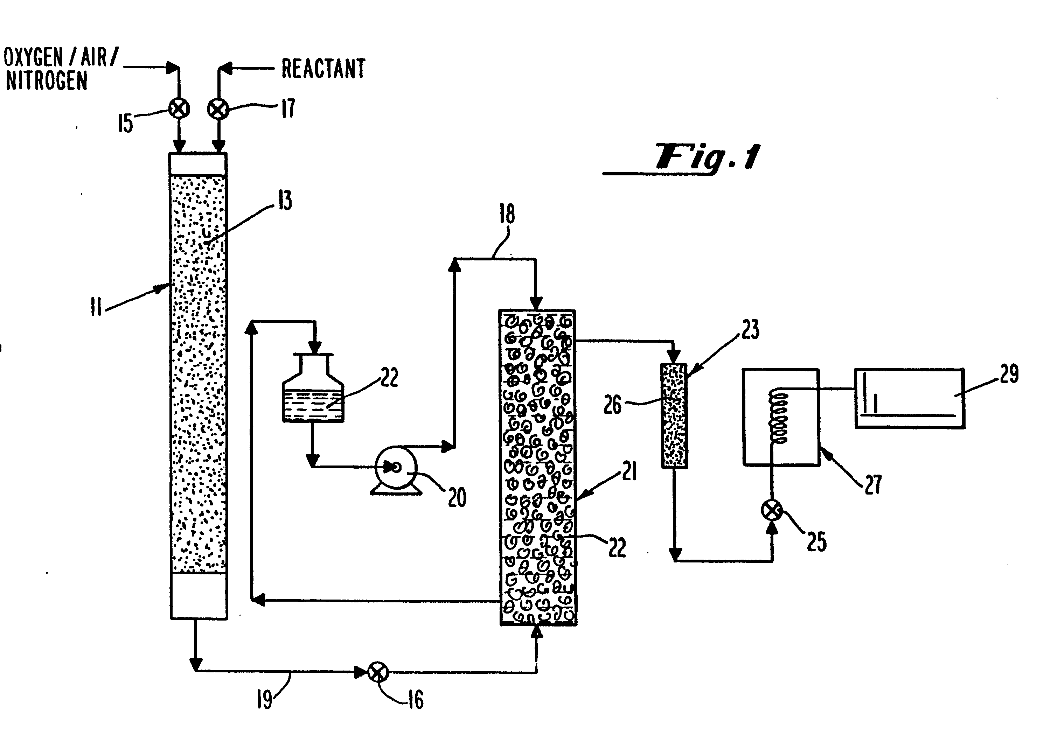

Turning now to Figure 1, reactor 11 is packed with catalyst 13. For example, $CrF_3 \cdot 3.5\ H_2O$ powder is placed in reactor 11 and is treated by initially passing a hot oxygen gas through the reactor, which is heated to from about 120°C to 600°C, for about 1 to 5 hours to dehydrate the catalyst which treatment is optionally followed by passing a mixture of nitrogen or other inert gas and HF (from about 50 to 80 mol% HF in nitrogen) through the reactor at a temperature of from 400° to 450°C for from 2 to 4 hours. The reactant to be dehydrofluorinated such as 1,1,1-trifluoroethane, preferably in a carrier gas such as nitrogen, is then passed through reactor 11 at a temperature of above 430°C to 600°C and preferably 500°C which provides 100% conversion of the reactant. Other inert carrier gases can be used such as helium or argon. Reactants which can be dehydrofluorinated by the process of the invention are fluorocarbons of the formula $RCH_2\text{-}CF_3$ where R is selected from hydrogen, methyl, isopropyl, t-butyl, cyclohexyl, vinyl, allyl, and phenyl. In the case of 1,1,1-trifluoroethane (143a) the reactant is dehydrofluorinated in accordance with the following reaction :

$$CH_3CF_3 \xrightarrow[\text{CrCat.}]{\Delta} CH_2{=}CF_2\ +\ HF$$

The use of an inert carrier gas is not necessary but is preferred because this acts to dilute the reaction products and minimize recombination of the reaction products as the reaction is reversible. It also extends the reaction time before catalyst regeneration is required. Mole ratios of carrier gas to reactant of up to about 5/1 are suitable with a preferred ratio being at least about 3/1. The components of the mixture can be fed individually through inlet valves 15 and 17 or the components can be premixed. Flow rates which provide a contact time with the catalyst of from about 0.1 to 0.4 minute are sufficient to produce 100% conversions. Longer times could be used but are unnecessary. The reaction products are removed at the bottom of reactor 11 through line 19 and valve 16 and are then passed up through scrubbing tower 21 countercurrent to a liquid alkaline stream 22 of, for example, aqueous 1.0 to 5.0 normal potassium hydroxide which is circulated through line 18 by pump 20 to remove the HF. Other aqueous hydroxides such as sodium or calcium hydroxide can also be used. The product 1,1-difluoroalkene is then passed through a drying tower 23 which is packed with a drying agent 26 such as anhydrous calcium sulfate. The conversion is periodically checked by passing product through valve 25 to a gas chromatograph 27 equipped with a recorder 29. When the conversion falls below a certain value, for example 50-60%, the flow of reactant and carrier gas is stopped and the catalyst is regenerated by passing a hot oxygen gas at a temperature of from 400° to 600°C through the reactor 11. By an oxygen gas is meant substantially pure oxygen, air or a synthetic mixture of molecular oxygen with mostly inert gases. Substantially pure oxygen is preferred to speed up the process.

Typically, the process produces 100% conversion of reactant to product for 1-2 hours and the catalyst can be regenerated in $\frac{1}{2}$ to 1 hour. After regeneration, the conversion returns to 100%.

The exact cause of the drop in conversion is not known but it may be caused by carbon formation on the surface of the catalyst and/or reaction of the catalyst with HF.

In contrast to the results reported in JP-A-54-130507 which discloses the catalysts as being chromium III compounds which are almost unchanged by exposure to HF during the process, analysis of the catalyst composition indicates that chromium oxyfluorides are formed during the hot oxygen treatment which are believed to provide the desired catalytic activity, for example, the results of the quantitative analysis for chromium and fluorine of reactivated catalyst in the dehydrofluorination of 1,1,1-trifluoroethane are consistent with the empirical formula $Cr_2O_5F_5$. This indicates that a transformation of Cr(III) fluoride to approximately a Cr(VI), oxyfluoride occurs. It is expected that the $CrO_2F_2$ oxyfluoride which is stable at elevated temperatures, could also be used as a catalyst.

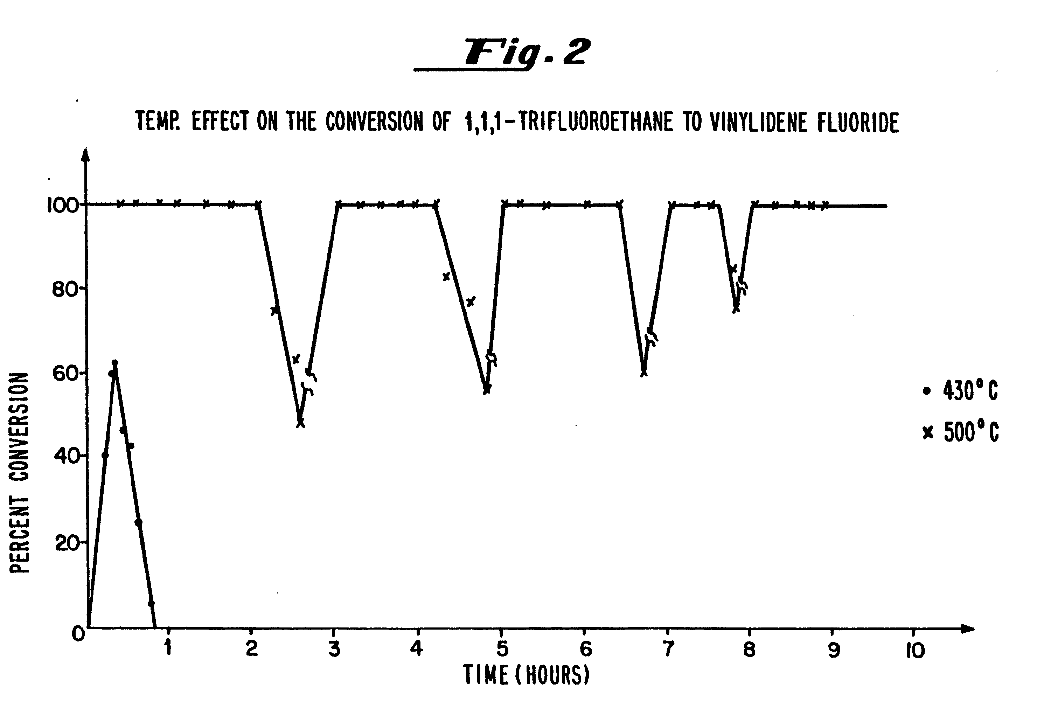

As an example of the process of the invention, 400 g of $CrF_3·3.5\ H_2O$ were placed in reactor 11 and the catalyst was activated with a flow of 165 ml per minute of hot air at 500°C for about 2 hours followed by feeding a mixture of nitrogen (165 ml) and HF(0.8 g/min) through the bed for about 2 hours. Nitrogen (165 ml) and 1,1,1-trifluoroethane (50 ml), (a mole ratio of 3.3/1), were fed into the reactor which was at a temperature of 500°C. The contact time was 0.1 minute. The product gas stream was washed with a stream of 4.0 N KOH solution to remove the HF, dried in the drying tower ($CaSO_4$) and periodically sampled to the gas chromatograph. As shown in the graph, the conversion remained at 100% for about two hours and then fell to about 50% after two and $\frac{1}{2}$ hours. The catalyst was then regenerated by passing 165 ccm of hot air through the reactor at a temperature of about 500°C for about an hour. Analysis of the regenerated catalyst indicated that it was converted to a chromium oxyfluoride. This transformation may have occurred either during the activation process (US-A-2,745,886) or the regeneration cycle. After regeneration and resumption of the reaction, the conversion returned to 100%. The reaction was continued until regeneration was again needed. The time between regenerations varied from 1 to 2 hours. Several reaction-regeneration cycles are illustrated by the 500°C line in the graph of Figure 2. The cycles were continued for a total of 110 hours without evidence of any deterioration in the ability to regenerate the catalyst.

In contrast to the process of the invention, when the process was carried out at a temperature of only 430°C, the conversions illustrated in the 430°C line in the graph of Figure 2 were obtained. The maximum conversion was about 60% and this conversion was maintained for only a few minutes. This illustrates that the reaction temperature as well as catalyst regeneration is important in obtaining the high conversions for extended times which are achieved by the process of the invention.

## Claims

1. A process for making a 1,1-difluoroalkene, characterized by flowing a gaseous 1,1,1-trifluoroalkane of the formula $RCH_2\text{-}CF_3$, where R is selected from hydrogen, methyl, isopropyl, t-butyl, cyclohexyl, vinyl, allyl and phenyl, through a catalyst bed which includes an inorganic chromium oxyfluoride obtained by treating chromium trifluoride hydrate powder with oxygen gas at a temperature of 120° to 600°C for 1 to 5 hours and optionally with hydrogen fluoride at a temperature of 400 to 450°C for 2 to 4 hours, at a reaction temperature above 430°C to form a 1,1-difluoroalkene of the formula $RCH=CF_2$ and HF as reaction products, and periodically reactivating the catalyst by interrupting the flow of 1,1,1-trifluoroalkane and passing an oxygen gas through said catalyst bed at a temperature of from 400 to 600°C for at least about 1/2 hour.

2. The process of claim 1 wherein the 1,1,1-trifluoroalkane is mixed with a carrier gas.

3. The process of claim 2 wherein the carrier gas is present in a mole ratio of up to about 5/1 inert gas to 1,1,1-trifluoroalkane.

4. The process of claim 3 wherein said mole ratio is at least about 3/1.

5. The process of claim 2 wherein said inert gas is nitrogen.

6. The process of claim 1 wherein the 1,1,1-trifluoroalkane is reacted in the absence of a carrier gas.

7. The process of claim 1 wherein the reaction temperature is at least about 500°C.

8. The process of claim 7 wherein the reaction temperature is from about 500 to 600°C.

9. The process of claim 2 wherein R is hydrogen, the inert carrier gas is nitrogen and the reaction temperature is about 500°C.

10. The process of claim 1 including the step of separating the HF from the 1,1-difluoroalkene product.

11. The process of claim 9 including the step of separating the HF from the 1,1-difluoroethylene product.

12. The process of claim 1 wherein the catalyst bed is formed by passing an oxygen gas at a temperature of from 120 to 600°C through a bed of $CrF_3 \cdot 3.5H_2O$ for from 1 to 5 hours and then passing a mixture of nitrogen or other inert gas through the bed at a temperature of from 400 to 450°C for from 2 to 4 hours.

13. The process of claim 1 wherein the catalyst bed includes $Cr_2O_5F_5$.

14. The process of claim 1 wherein the catalyst bed includes $CrO_2F_2$.

15. The process of claim 9 wherein the HF is separated from the 1,1-difluoroethylene product by contacting the reaction products with an aqueous, alkaline solution.

16. The process of claim 1, characterized in that for making 1,1-difluoroethylene gaseous 1,1,1-trifluoroethane mixed with nitrogen in a ratio of from about 3/1 to 5/1 nitrogen to 1,1,1-trifluoroethane is flown through a catalyst bed which includes an inorganic chromium oxyfluoride at a reaction temperature of about 500 to 600°C so as to dehydrofluorinate the 1,1,1-trifluoroethane and form 1,1-difluoroethylene and HF as reaction products, the reaction products are separated by contacting them with an aqueous, alkaline solution to remove the HF, and the catalyst is periodically reactivated by interrupting the flow of 1,1,1-trifluoroethane and nitrogen and passing an oxygen gas through said catalyst bed at a temperature of about 400 to 600°C for from about 1/2 to 1 hour.

17. The process of claim 16 wherein the oxygen gas is substantially pure oxygen.

18. The process of claim 16 wherein the reaction and regeneration temperatures are each about 500°C.

## Ansprüche

1. Verfahren zur Herstellung eines 1,1-Difluoralkens, dadurch **gekennzeichnet**, daß man ein gasförmiges 1,1,1-Trifluoralkan der Formel $RCH_2$-$CF_3$, worin R aus Wasserstoff, Methyl, Isopropyl, t-Butyl, Cyclohexyl, Vinyl, Allyl und Phenyl ausgewählt ist, durch ein Katalysatorbett, das ein anorganisches Chromoxyfluorid, erhalten durch Behandlung von Chromtrifluorid-hydrat-Pulver mit Sauerstoffgas bei einer Temperatur von 120°C bis 600°C über 1 bis 5 Stunden und gegebenenfalls mit Fluorwasserstoff bei einer Temperatur von 400°C bis 450°C über 2 bis 4 Stunden, enthält, bei einer Reaktionstemperatur oberhalb 430°C strömen läßt, um ein 1,1-Difluoralken der Formel $RCH=CF_2$ und HF als Reaktionsprodukte zu erhalten, und daß man periodisch den Katalysator reaktiviert, indem man den Strom des 1,1,1-Trifluoralkans unterbricht und Sauerstoffgas durch das genannte Katalysatorbett bei einer Temperatur von 400°C bis 600°C über mindestens etwa 1/2 Stunde leitet.

2. Verfahren nach Anspruch 1, dadurch **gekennzeichnet**, daß man das 1,1,1-Trifluoralkan mit einem Trägergas mischt.

3. Verfahren nach Anspruch 2, dadurch **gekennzeichnet**, daß das Trägergas in einem Molverhältnis von bis zu etwa 5 : 1 Inertgas zu 1,1,1-Trifluoralkan vorhanden ist.

4. Verfahren nach Anspruch 3, dadurch **gekennzeichnet**, daß das genannte Molverhältnis mindestens etwa 3 : 1 ist.

5. Verfahren nach Anspruch 2, dadurch **gekennzeichnet**, daß das genannte Inertgas Stickstoff ist.

6. Verfahren nach Anspruch 1, dadurch **gekennzeichnet**, daß man das 1,1,1-Trifluoralkan in Abwesenheit eines Trägergases umsetzt.

7. Verfahren nach Anspruch 1, dadurch **gekennzeichnet**, daß die Reaktionstemperatur mindestens etwa 500°C ist.

8. Verfahren nach Anspruch 7, dadurch **gekennzeichnet**, daß die Reaktionstemperatur etwa 500 bis 600°C ist.

9. Verfahren nach Anspruch 2, dadurch **gekennzeichnet**, daß R für Wasserstoff steht, daß das inerte Trägergas Stickstoff ist und daß die Reaktionstemperatur etwa 500°C beträgt.

10. Verfahren nach Anspruch 1, dadurch **gekennzeichnet**, daß es die Stufe der Abtrennung des HF von dem 1,1-Difluoralken-Produkt einschließt.

11. Verfahren nach Anspruch 9, dadurch **gekennzeichnet**, daß es die Stufe der Abtrennung des HF von dem 1,1-Difluorethylen-Produkt einschließt.

12. Verfahren nach Anspruch 1, dadurch **gekennzeichnet**, daß man das Katalysatorbett dadurch bildet, daß man Sauerstoffgas bei einer Temperatur von 120 bis 600°C durch ein Bett von $CrF_3 \cdot 3,5H_2O$ über 1 bis 5 Stunden leitet und sodann ein Gemisch aus Stickstoff oder einem anderen Inertgas durch das Bett bei einer Temperatur von 400 bis 450°C über 2 bis 4 Stunden leitet.

13. Verfahren nach Anspruch 1, dadurch **gekennzeichnet**, daß das Katalysatorbett $Cr_2O_5F_5$ enthält.

14. Verfahren nach Anspruch 1, dadurch **gekennzeichnet**, daß das Katalysatorbett $CrO_2F_2$ enthält.

15. Verfahren nach Anspruch 9, dadurch **gekennzeichnet**, daß man den HF aus dem 1,1-Difluorethylen-Produkt durch Kontaktierung der Reaktionsprodukte mit einer wäßrigen alkalischen Lösung abtrennt.

16. Verfahren nach Anspruch 1, dadurch gekennzeichnet, daß man zur Herstellung von 1,1-Difluorethylen gasförmiges 1,1,1-Trifluorethan, das mit Stickstoff in einem Verhältnis von etwa 3:1 bis 5:1 Stickstoff zu 1,1,1-Trifluorethan vermischt worden ist, durch ein Katalysatorbett, das ein anorganisches Chromoxyfluorid enthält, bei einer Reaktionstemperatur von etwa 500 bis 600°C strömen läßt, um das 1,1,1-Trifluorethan zu dehydrofluorieren und 1,1-Difluorethylen und HF als Reaktionsprodukte zu bilden, daß man die Reaktionsprodukte trennt, indem man sie mit einer wäßrigen alkalischen Lösung zur Entfernung des HF kontaktiert, und daß man den Katalysator periodisch reaktiviert, indem man den Strom von 1,1,1-Trifluorethan und Stickstoff unterbricht und Sauerstoffgas durch das genannte Katalysatorbett bei einer Temperatur von etwa 400 bis 600°C über etwa 1/2 bis 1 Stunde leitet.

17. Verfahren nach Anspruch 16, dadurch gekennzeichnet, daß das Sauerstoffgas im wesentlichen reiner Sauerstoff ist.

18. Verfahren nach Anspruch 16, dadurch gekennzeichnet, daß die Reaktions- und Regenerierungstemperaturen jeweils etwa 500°C sind.

## Revendications

1. Procédé de production d'un 1,1-difluoralcène, caractérisé par le passage d'un courant gazeux de 1,1,1-trifluoralcane de formule $RCH_2$-$CF_3$, dans laquelle R est choisi entre l'hydrogène et les radicaux méthyle, isopropyle, tertiobutyle, cyclohexyle, vinyle, allyle et phényle, à travers un lit de catalyseur qui contient un oxyfluorure de chrome inorganique obtenu par traitement de trifluorure de chrome hydraté en poudre avec de l'oxygène gazeux à une température de 120 à 600°C pendant 1 à 5 heures et, à titre facultatif, avec du fluorure d'hydrogène à une température de 400 à 450°C pendant 2 à 4 heures, à une température de réaction au-dessus de 430°C pour former un 1,1-difluoralcène de formule $RCH$=$CF_2$ et du HF comme produits de réaction, et la réactivation périodique du catalyseur par interruption du courant de 1,1,1trifluoralcane et passage d'oxygène gazeux à travers le lit de catalyseur à une température de 400 à 600°C pendant au moins environ 1/2 heure.

2. Procédé suivant la revendication 1, dans lequel le 1,1,1-trifluoralcane est mélangé avec un gaz d'entraînement.

3. Procédé suivant la revendication 2, dans lequel le gaz d'entraînement est présent dans un rapport molaire du gaz inerte au 1, 1, 1-trifluoralcane allant jusqu'à environ 5/1.

4. Procédé suivant la revendication 3, dans lequel le rapport molaire est d'au moins environ 3/1.

5. Procédé suivant la revendication 2, dans lequel le gaz inerte est l'azote.

6. Procédé suivant la revendication 1, dans lequel le 1,1,1-trifluoralcane est amené à réagir en l'absence d'un gaz d'entraînement.

7. Procédé suivant la revendication 1, dans lequel la température de réaction est d'au moins environ 500°C.

8. Procédé suivant la revendication 7, dans lequel la température de réaction va d'environ 500 à 600°C.

9. Procédé suivant la revendication 2, dans lequel R est l'hydrogène, le gaz inerte d'entraînement est l'azote et la température de réaction est d'environ 500°C.

10. Procédé suivant la revendication 1, comprenant l'étape de séparation du HF du 1,1-difluoralcène obtenu comme produit.

11. Procédé suivant la revendication 9, comprenant l'étape de séparation du HF du 1,1,-difluoréthylène obtenu comme produit.

12. Procédé suivant la revendication 1, dans lequel le lit de catalyseur est formé par passage d'oxygène gazeux à une température d'environ 120 à 600°C à travers un lit de $CrF\cdot3,5H_2O$ pendant 1 à 5 heures, puis passage d'un mélange d'azote ou d'un autre gaz inerte à travers le lit à une température de 400 à 450°C pendant 2 à 4 heures.

13. Procédé suivant la revendication 1, dans lequel le lit de catalyseur contient du $Cr_2O_5F_5$.

14. Procédé suivant la revendication 1, dans lequel le lit de catalyseur contient du $CrO_2F_2$.

15. Procédé suivant la revendication 9, dans lequel le HF est séparé du 1,1-difluoréthylène obtenu comme produit par mise en contact des produits de réaction avec une solution aqueuse alcaline.

16. Procédé suivant la revendication 1, caractérisé en ce que, pour produire du 1,1-difluoréthylène, on fait passer un courant de 1,1,1-trifluoréthane gazeux mélangé à de l'azote dans un rapport de l'azote au 1,1,1-trifluoréthane d'environ 3/1 à 5/1 à travers un lit de catalyseur qui contient un oxyfluorure de chrome inorganique à une température de réaction d'environ 500 à 600°C de manière à déshydrofluorer le 1,1,1-trifluoréthane et à former du 1,1-difluoréthylène et du HF comme produits de réaction, on sépare les produits de réaction par leur mise en contact avec une solution aqueuse alcaline pour éliminer le HF et on réactive périodiquement le catalyseur en interrompant le courant de 1,1,1-trifluoréthane et d'azote et en faisant passer un courant d'azote gazeux à travers le lit du catalyseur à une température d'environ 400 à 600°C pendant environ 0,5 à 1 heure.

17. Procédé suivant la revendication 16, dans lequel l'oxygène gazeux est de l'oxygène pratiquement pur.

18. Procédé suivant la revendication 16, dans lequel les températures de réaction et de régénération sont chacune d'environ 500°C.

OXYGEN / AIR /
NITROGEN

REACTANT

15

17

*Fig.1*

13

18

11

23

22

26

29

20

21

22

27

25

19

16

EP 0 234 002 B1

# *Fig. 2*

**TEMP. EFFECT ON THE CONVERSION OF 1,1,1-TRIFLUOROETHANE TO VINYLIDENE FLUORIDE**

• 430°C
× 500°C

PERCENT CONVERSION %

TIME (HOURS)

EP 0 234 002 B1